Europäisches Patentamt

(19) European Patent Office

Office européen des brevets

(11) Veröffentlichungsnummer: **0 322 350**
A1

(12) **EUROPÄISCHE PATENTANMELDUNG**

(21) Anmeldenummer: 88730269.3

(22) Anmeldetag: 02.12.88

(51) Int. Cl.[4]: **A 61 K 49/00**
A 61 B 8/08

(30) Priorität: 02.12.87 DE 3741199

(43) Veröffentlichungstag der Anmeldung:
28.06.89 Patentblatt 89/26

(84) Benannte Vertragsstaaten: **ES GR**

(71) Anmelder: **SCHERING AKTIENGESELLSCHAFT Berlin
und Bergkamen
Müllerstrasse 170/178 Postfach 65 03 11
D-1000 Berlin 65 (DE)**

(72) Erfinder: **Fritzsch, Thomas, Dr.
Elisenstrasse 2
D-1000 Berlin 41 (DE)**

**Siegert, Joachim, Dr.
Weddingstrasse 5
D-1000 Berlin 65 (DE)**

**Uhlendorf, Volkmar, Dr.
Eichenallee 30
D-1000 Berlin 19 (DE)**

**Schlief, Reinhard, Dr.
Neue Strasse 21
D-1000 Berlin 37 (DE)**

**Niendorf, Hans Peter, Dr.
Wernerstrasse 5
D-1000 Berlin 33 (DE)**

**Cramer, Eckehart, Dr. Dipl.-Phys.
Berliner Strasse 79
D-1000 Berlin 37 (DE)**

**Poland, Hans, Dr.
Spirdingseestrasse 29
D-1000 Berlin 19 (DE)**

**Ganter, Doris, Dipl.-Ing.
Steinbergstrasse 37
D-8034 Germering (DE)**

(74) Vertreter: **Maikowski, Michael, Dipl.-Ing. Dr.
Xantener Strasse 10
D-1000 Berlin 15 (DE)**

(54) **Verwendung von bläschenhaltigen Medien für die Stosswellen- und Ultraschalltherapie.**

(57) Die Erfindung betrifft die Verwendung von Suspensionen oder Lösungen mit Mikrobläschen für die Stoßwellen- und Ultraschalltherapie. Bei der Verwendung der Mittel wird zur Entlastung des Patienten die Lithotripsie mittels Einstrahlung von Stoß- und Ultraschallwellen mit einer geringeren Gesamtenergie als ohne Verwendung des Mittels erreicht.

EP 0 322 350 A1

## Beschreibung

### Verwendung von bläschenhaltigen Medien für die Stoßwellen- und Ultraschalltherapie

Die Erfindung betrifft die Verwendung von bläschenhaltigen Medien für die Stoßwellen- und Ultraschalltherapie.

Eine wirkungsvolle Steinzerstörung ist die elektrohydraulische Steinzerstörung, bei der eine Druckwelle außerhalb des Körpers erzeugt und durch das Körpergewebe hindurch auf den Stein fokussiert wird. Bekanntlich werden derartige Wellen in Flüssigkeit durch Anregung einer Funkenstrecke mit gedämpftem Schwingkreis oder mit Kondensatorentladung erzeugt. Die Funkenstrecke ist von einem fokusierenden Reflektor umgeben.

Andere Methoden der Wellenerzeugung sind z.B. gepulste Laser, piezoelektrische oder elektromagnetische Elemente. Die Fokussierung der Wellen kann dabei außer durch Reflektion an einem Halbelipsoid durch parabolische Anordnung der Energiequellen oder durch Bündelung mittels einer akustischen Linse erfolgen (F. Eisenberger et al. Urologische Steintherapie, Georg Thieme Verlag Stuttgart 1987 S. 25 ff. und die aufgeführten Literaturstellen).

Bei den bekannten Verfahren werden Spannungen bis max. 26 kV und etwa 3.000 Stoßwellen pro Behandlung angewandt. Die durchschnittliche Behandlungsdauer beträgt 40 Minuten. Da die Stoßwellen vom Patienten als starker Schlag gegen den Rücken empfunden werden, ist Anästhesie erforderlich. Bei der Notwendigkeit des Einsatzes von Wellen hoher Energie wird sogar Vollnarkose angewandt, ansonsten verschiedene Methoden der Teilanästhesie (F. Eisenberger et al. a.a.O.).

Als Wellen für die Therapie finden solche elastomechanischen Schwingungen Anwendung, die als Stoß- oder Ultraschallwellen bezeichnet werden.

Der Erfindung liegt die Aufgabe zugrunde, für die Therapie mittels Stoßwellen und Ultraschall ein Mittel zu verwenden, mit dem durch eine Wirkungsverstärkung am Ort der Steinzertrümmerung zur Entlastung des Patienten die eingestrahlte Gesamtenergie pro Behandlung verglichen mit den bekannten Verfahren, verringert werden kann.

Überraschenderweise hat sich gezeigt, daß bei der Stoßwellen- und Ultraschalltherapie mittels Bubble-Suspensionen die Lithotripsie von Steinen erleichtert wird, wenn die Mikrobläschen vorab nahe an die Konkremente herangebracht werden.

In vorteilhafter Weise können

1. eine Supension mit Mikrobläschen bestehend aus Mikropartikeln aus einer grenzflächenaktiven Substanz in einem flüssigen Träger oder

2. eine Suspension mit Mikrobläschen bestehend aus Mikropartikeln aus einem nicht grenzflächenaktiven Feststoff in einem flüssigen Träger oder

3. eine Suspension mit Mikrobläschen bestehend aus Mikropartikeln aus einer Mischung aus mindestens einer grenzflächenaktiven Substanz mit einem nicht grenzflächenaktiven Feststoff in einem flüssigen Träger für die Stoßwellen- und Ultraschalltherapie Verwendung finden.

Einige dieser erfindungsgemäß verwendeten Mittel werden bereits in den EP-OS 122 624 und 123 235 beschrieben.

Mit Vorteil kann das verwendete Mittel Mikropartikel enthalten, die als grenzflächenaktive Substanz Lecithine, Polyoxyethylenfettsäureester, Glycerinpolyethylenglykolrizinoleat, Polyoxyethylenpolyoxypropylen-Polymere Saccharoseester, Xyloglyceride, gesättigte oder ungesättigte (C4-C20)-Fettalkohole, gesättigte oder ungesättigte (C4-C20)-Fettsäuren oder deren Salze, Mono-, Di- und Triglyceride, Fettsäureester als Mikropartikel enthalten. Ferner ist es möglich, daß das Mittel Mikropartikel enthält, die als grenzflächenaktiven Stoff Sojaölsaccharoseglycerid oder Polyethylenglykolsorbitanmonostearat enthalten.

Mit besonderem Vorteil können als Mikropartikel Magnesiumstearat, Ascorbylpalmitat, Saccharosemonopalmitat, Saccharosemonostearat, Saccharosedistearat oder Butylstearat als grenzflächenaktive Substanzen im Mittel enthalten sein.

Die Suspension mit Mikrobläschen enthält die grenzflächenaktive Substanz in einer Konzentration von 0,001 bis 5 Gewichtsprozenten, vorzugsweise von 0,04 bis 1 Gewichtsprozent.

Als nicht grenzflächenaktive Feststoffe kann das Mittel mit Vorzug Cyclodextrine, Monosaccharide, Disaccharide, Trisaccharide, Polyole oder anorganische oder organische Salze mit einer Konzentration von 2 bis 50 Gewichtsprozenten, vorzugsweise von 9 bis 40 Gewichtsprozenten enthalten. Das Mittel kann ferner Mikropartikel enthalten, die als nicht grenzflächenaktiven Feststoff Dextrose, Maltose, Galactose, Lactose oder α-Cyclodextrin in einer Konzentration von 2 bis 50 Gewichtsprozenten, vorzugsweise 9 bis 40 Gewichtsprozenten, enthalten.

Als geeignete anorganische oder organische Salze sind Natriumchlorid, Natriumcitrat, Natriumacetat oder Natriumtartrat zu nennen.

Mit Vorteil kann das verwendete Mittel als physiologisch verträglichen flüssigen Träger Wasser, physiologische Elektrolytlösung, wäßrige Lösung von ein- oder mehrwertigen Alkoholen oder Polyetheralkoholen oder wäßrige Lösung eines Mono- oder Disaccharids oder Ringer-Lösung oder Tyrode-Lösung oder eine wäßrige Lösung von Maltose, Dextrose, Lactose oder Galactose enthalten. Mit besonderem Vorteil kann der flüssige Träger Glycerin, Polyethylenglycol oder Propylenglykolmethylether enthalten.

Als physiologisch verträglicher flüssiger Träger kann aber auch physiologische Kochsalzlösung in dem Mittel enthalten sein.

Überraschenderweise wurde weiter gefunden, daß ein erfindungsgemäß verwendetes Mittel, welches Mikropartikel aus Maltose, Dextrose, Lactose oder Galactose in einem flüssigen Träger der Wasser, eine physiologische Elektrolytlösung wie 0,9 %ige wäßrige Natriumchloridlösung, Ringer-Lösung oder Tyrode-Lösung oder eine wäßrige Lösung von Maltose, Dextrose, Lactose oder Galaktose sein kann, enthält, ohne Zusatz von viskositätserhöhenden Stoffen wie beispielsweise Propylenglykol eine gute Wirkungsverstärkung bei der Stoßwellen- und Ultraschalltherapie ermöglicht.

Derartige, erfindungsgemäß verwendete Mittel werden in der EP-OS 131 540 beschrieben.

Dabei kann das erfindungsgemäß benutzte Mittel Mikropartikel aus Lactose in bis zu 25 %iger (Gewichtsprozent) wäßriger Lactose-Lösung enthalten. Insbesondere können die erfindungsgemäß verwendeten Mittel auch Mikropartikel aus Galaktose in bis zu 20 %iger wäßriger Galaktose-Lösung oder Mikropartikel aus Galaktose in Wasser enthalten.

Es liegt im Rahmen der Erfindung, daß das Mittel Mikropartikel einer Mischung von Butylstearat und Galactose in Wasser oder eine Mischung von Sojaölsaccharose glycerid und Galactose in Wasser oder Polyethylenglycolsorbitanmonostearat und Galactose in physiologischer Kochsalzlösung oder Ölsäure und Galactose in physiologischer Kochsalzlösung enthält.

Es liegt ferner im Rahmen der Erfindung, daß ein erfindungsgemäß verwendetes Mittel eine flüssige Lösung mit Mikrobläschen ist, bestehend aus der Mischung von 0,01 bis 10 Gewichtsprozenten eines Tensides oder Tensidgemisches mit einer wäßrigen oder mit Wasser mischbaren Trägerflüssigkeit und der Mischung von 0,5 bis 50 Gewichtsprozenten einer viskositätserhöhenden Substanz oder eines Substanzgemisches in einer wäßrigen oder mit Wasser mischbaren Trägerflüssigkeit, wobei beide Mischungen getrennt oder vereinigt vorliegen.

Mit besonderem Vorteil kann ein Mittel für die Stoßwellen- und Ultraschalltherapie benutzt werden, bestehend aus einer Mischung von 0,01 bis 10 Gewichtsprozenten eines Tensides oder Tensidgemisches in einer wäßrigen oder mit Wasser mischbaren Trägerflüssigkeit, die 0,05 bis 5 Gewichtsprozente eines physiologisch verträglichen carbonsauren Salzes enthält und der Mischung von 0,5 bis 50 Gewichtsprozenten einer viskositätserhöhenden Substanz oder eines Substanzgemisches mit einer wäßrigen oder mit Wasser mischbaren Trägerflüssigkeit, die eine dem carbonsauren Salz äquivalente Menge physiologisch verträglicher Säure enthält.

Als physiologisch verträgliches carbonsaures Salz findet besonders Natriumhydrogencarbonat und Kaliumhydrogen carbonat Anwendung. Als physiologisch verträgliche Säuren sind besonders Milchsäure, Zitronensäure und Brenztraubensäure zu nennen.

Derartig erfindungsgemäß verwendete Mittel werden in der EP-PS 0077 752 beschrieben.

Als Tenside sind sowohl ionogene als auch nichtionogene Tenside, die gleichzeitig auch viskositätserhöhend wirken können, geeignet. Als nichtionogene Tenside seien genannt: Lecithine, Lecithinfraktionen und deren Abwandlungsprodukte, Polyoxyethylenfettsäureester wie Polyoxyethylenfettalkoholäther, polyoxyethylierte Sorbitanfettsäureester, Glycerin-polyethylenglykoloxystearat, Glycerinpolyethylenglykolrhizinoleat, ethoxylierte Sojastearine, ethoxylierte Rizinusöle und deren hydrierte Derivate, Cholesterol, Polyoxyethylenpolyoxypropylen-Polymere, wobei Polyoxyethylenfettsäurestearate und Polyoxyethylenpolyoxypropylen-Polymere mit dem Molgewicht 6800-8975, 13300 und 16250 bevorzugt sind.

Als ionogene Tenside kommen in Frage: Quarternäre Ammoniumbase, Natriumlaurylsulfat, Natriumdioctylsulfosuccinat

Als viskositätserhöhende Substanzen kommen in Frage Mono- oder Polysaccharide wie Glucose, Lävulose, Galactose, Lactose, Sorbit, Mannit, Xylit, Saccharose oder Dextrane, Cyclodextrine, Hydroxyethylstärke und Polyole. Als Polyole werden verwendet Glycerin, Polyglykole, Inulin und 1,2-Propandiol. Zur Viskositäterhöhung können weiterhin benutzt werden Proteine, proteinähnliche Stoffe, Aminosäuren oder Blutersatzstoffe wie beispielsweise Plasmaproteine, Gelatine, Oxypolygelatine und Gelatinederivate oder deren Gemische.

Die Konzentration dieser genannten Stoffe in der Lösung kann 0,5 bis 50 Gewichtsprozente betragen, wobei die Höchstkonzentration auch vom gelösten Stoff abhängt. So können beispielsweise Glucose oder Lactose mit einer Konzentration von 0,5 bis 50 Gewichtsprozenten verwendet werden, wogegen Gelatine eine bevorzugte Konzentration von 0,5 bis 2 Gewichtsprozenten hat. Die Oxypolygelatine wird bevorzugt mit einer Konzentration von 0,5 bis 10 Gewichtsprozenten eingesetzt.

Man kann auch Tenside verwenden, die gleichzeitig viskositätserhöhend wirken wie beispielsweise Polyoxyethylenpolyoxypropylen-Polymere mit dem Molekulargewicht von 4750 bis 16250.

In diesem Fall beträgt die Konzentration der Tenside mit viskositätserhöhender Wirkung 1 bis 20 Gewichtsprozente, vorzugsweise 3 bis 10 Gewichtsprozente. Das Tensid oder Tensidgemisch wird vorzugsweise in Gegenwart des viskositätserhöhenden Stoffes oder Stoffgemische in einer Trägerflüssigkeit gelöst.

Als Trägerflüssigkeit kann Wasser verwendet werden oder wäßrige Lösungen, die physiologisch verträglich sind wie beispielsweise physiologische Elektrolytlösungen wie physiologische Kochsalzlösung, mit Wasser mischbare ein- oder mehrwertige Alkohole, Ringerlösung, Tyrodelösung oder die wäßrigen Lösungen von Natriumchlorid, Calciumchlorid Natriumhydrogencarbonat, Natriumcitrat Natriumacetat oder Natriumtartrat oder Salzlösungen, wie sie üblicherweise als Infusionslösungen verwendet werden, oder deren Gemische.

Die vorbezeichneten Mittel finden Anwendung bei jeglicher Form der extrakorporalen Stoßwellenlithotripsie (ESWL). Sie eignen sich in hervorragender Art und Weise zur Verstärkung bei der Zertrümmerung von Nierensowie Gallensteinen.

Die Mittel werden durch retrograde oder antegrade Applikation in die Zielorgane transportiert.

Es hat sich herausgestellt, daß beispielsweise die folgenden drei Methoden für die Durchführung der ESWL vorteilhaft sind:

    a) Wird das Mittel retrograd in die Niere eingebracht, so ist zuvor über einen Katheter, der über die Harnleiter in die Niere eingebracht wird, der Harn abzuziehen. Nach Harnabzug wird das Mittel (2 - 50 ml abhängig vom Nierenbeckenvolumen) in die Niere eingebracht, um nach einer kurzen Verweilzeit von ca. 0,5 bis 2 Minuten wieder aus der Niere entfernt zu werden. Anschließend wird die Niere mit physiologisch verträglicher Lösung gefüllt. Die Bestrahlung mittels elastomechanischer Schwingungen (Stoßwellen, Ultraschallwellen) zum Zwecke der Steinzertrümmerung kann sich nun anschließen.

b) Mittels eines Endoskops wird beispielsweise bei retrograder Arbeitsweise das Mittel in unmittelbare Steinnähe gebracht. Die Steinoberfläche wird gezielt mit der Mittellösung (Volumen: 0,1 - 5 ml) versehen. Der Stein wird gezielt mit der Mittellösung umhüllt (Volumen der applizierten Lösung: 1 - 5 ml) Die Bestrahlung durch elastomechanische Schwingungen kann sich nun anschließen.

c) Bei antegrader Applikation wird das Mittel per Punktionsnadel in die Niere eingebracht und nach einer kurzen Verweildauer (0,5 - 2 Minuten) wieder entfernt und dann den Schwingungen ausgesetzt.

Es ist darauf zu achten, um den gewünschten Erfolg der Lithotripsie mit Hilfe der beschriebenen Mittel zu erreichen, daß die Hohlräume der Zielorgane (beispielsweise Niere, Galle) nicht vollständig mit dem jeweiligen Mittel befüllt werden. Bei Anwesenheit einer zu großen Anzahl von Mikrobläschen in den Zielorganen, bleibt der gewünschte Erfolg der Lithotripsie aus. Die zahlreichen Mikrobläschen absorbieren und streuen an den Steinen die eingestrahlte elastomechanische Schwingung. Die eingestrahlten Wellen gelangen somit nicht mit ausreichender Energie zum Zielort, dem Stein.

Die Eigenschaften der Mittel bewirken, daß bei einer Vorgehensweise wie unter a) geschildert, die Mikrobläschen des Mittels z.B. durch Adhäsionskräfte an der Ober fläche der Steine haften bleiben. Durch die anschließende Verdrängung mittels physiologisch verträglicher Lösung wird nur das Mittel aus dem Organraum verdrängt, die an den Steinen haftenden Mikrobläschen bleiben in ausreichender Menge haften. Es liegen dann Steine vor, die mit einer Mikrobläschenschicht umhüllt sind, und die sich (Stein + Bläschen) in der physiologisch verträglichen Lösung befinden. Werden nun die elastomechanischen Schwingungen eingestrahlt, wird die Energie dieser Schwingungen von den am Stein haftenden Bläschen aufgenommen und zur Steinzertrümmerung benutzt.

Die Therapie von Gallensteinen ist beispielsweise über antegrade bzw. retrograde Punktion mit Hilfe bekannter Methoden in analoger Weise möglich.

Beispiele:

1.: In einem Wasserbad, welches mit einem Funkenentladungsstoßwellenerzeuger versehen ist, wird in einem Probengefäß ein humaner Gallenstein in den zweiten Brennpunkt des Elipsoids eingebracht. Das Probengefäß wird dann mit dem Mittel (20 ml einer 25%igen Galactosesuspension in Wasser) gefüllt. Anschließend wird aus dem Probengefäß die Galactosesuspension mittels Stechheber entfernt und das Probengefäß mit 20 ml physiologischer Kochsalzlösung gefüllt. Das Konkrement im Probengefäß wird nun den Stoßwellen ausgesetzt. Nach nur neun Stößen zerfiel dieser Stein in viele kleine Bruchstücke.

2.: Der Versuchsaufbau gleicht dem in Beispiel 1. Der Gallenstein (gleiche Größe wie im Beispiel 1.) wurde jedoch nicht mit dem Galactosemittel behandelt, sondern sofort in die physiologische Kochsalzlösung eingebracht. Nachdem 100 Stöße der gleichen Energie auf den Stein einwirkten, wurde keine Steinzertrümmerung erreicht.

**Patentansprüche**

1. Verwendung einer Suspension mit Mikrobläschen bestehend aus Mikropartikeln aus einer grenzflächenaktiven Substanz in einem flüssigen Träger,
für die Stoßwellen- und Ultraschalltherapie.

2. Verwendung einer Suspension mit Mikrobläschen bestehend aus Mikropartikeln aus einem nicht grenzflächenaktiven Feststoff in einem flüssigen Träger,
für die Stoßwellen- und Ultraschalltherapie.

3. Verwendung einer Suspension mit Mikrobläschen bestehend aus Mikropartikeln aus einer Mischung aus mindestens einer grenzflächenaktiven Substanz mit einem nicht grenzflächenaktiven Feststoff in einem flüssigen Träger,
für die stoßwellen- und Ultraschalltherapie.

4. Verwendung eines Mittels nach einem der Ansprüche 1 oder 3,
enthaltend
als grenzflächenaktive Substanz Lecithine, Polyoxyethylenfettsäureester, Glycerinpolyethylenglykolrizinoleat, Polyoxyethylenpolyoxypropylen-Polymere, Saccharoseester, Xyloglyceride, gesättigte oder ungesättigte (C4-C20)-Fettalkohole, gesättigte oder ungesättigte (C4-C20)-Fettsäuren oder deren Salze, Mono-, Di- und Triglyceride, Fettsäureester, Sojaölsaccharoseglycerid oder Polyethylenglykolsorbitanmonostearat als Mikropartikel in einer Konzentration von 0,001 bis 10 Gewichtsprozenten vorzugsweise 0,04 bis 1 Gewichtsprozent.

5. Verwendung eines Mittels nach einem der Ansprüche 1 oder 3,
enthaltend
als grenzflächenaktive Substanz Magnesiumstearat, Ascorbylpalmitat, Saccharosemonopalmitat, Saccharosemonostearat, Saccharosedistearat oder Butylstearat in einer Konzentration von 0,001 bis 10 Gewichtsprozenten vorzugsweise 0,04 bis 1 Gewichsprozent.

6. Verwendung eines Mittels nach einem der Ansprüche 2 oder 3,
enthaltend

als nicht grenzflächenaktiven Feststoff Cyclodextrine, Monosaccharide, Disaccharide, Trisaccharide, Polyole oder anorganische oder organische Salze mit einer Konzentration von 2 bis 50 Gewichtsprozenten, vorzugsweise von 9 bis 40 Gewichtsprozenten.

7. Verwendung eines Mittels nach einem der Ansprüche 2 oder 3, enthaltend als nicht grenzflächenaktiven Feststoff Galactose, Dextrose, Maltose, Lactose oder α-Cyclodextrin in einer Konzentration von 2 bis 50 Gewichtsprozenten, vorzugsweise von 9 bis 40 Gewichtsprozenten.

8. Verwendung eines Mittels nach einem der Ansprüche 1-3, enthaltend als flüssigen Träger, der physiologisch verträglich ist, Wasser, physiologische Elektrolytlösung, wäßrige Lösung von ein- oder mehrwertigen Alkoholen oder Polyetheralkoholen oder der wäßrigen Lösung eines Mono- oder Disaccharides.

9. Verwendung eines Mittels nach einem der Ansprüche 1-3, enthaltend als flüssigen Träger Ringer-Lösung oder Tyrode-Lösung oder eine wäßrige Lösung von Maltose, Dextrose, Lactose oder Galactose.

10. Verwendung eines Mittels nach einem der Ansprüche 1-3, enthaltend als flüssigen Träger eine wäßrige Lösung von Glycerin, Polyethylenglykol oder Propylenglykolmethylether.

11. Verwendung eines Mittels nach Anspruch 3, enthaltend Mikropartikel einer Mischung aus Butylstearat und Galactose in Wasser.

12. Verwendung eines Mittels nach Anspruch 2, enthaltend Mikropartikel aus Galactose in Wasser.

13. Verwendung eines Mittels nach Anspruch 2, enthaltend Mikropartikel aus Galactose in bis 20 %iger (Gewichtsprozent) wäßriger Galactose-Lösung.

14. Verwendung eines Mittels nach Anspruch 3, enthaltend Mikropartikel einer Mischung aus Polyethylenglykolsorbitanmonostearat und Galactose in physiologischer Kochsalzlösung.

15. Verwendung eines Mittels nach Anspruch 3, enthaltend Mikropartikel einer Mischung aus Ölsäure und Galactose in physiologischer Kochsalzlösung.

16. Verwendung eines Mittels nach Anspruch 2, enthaltend Mikropartikel aus Lactose in bis zu 25 %iger (Gewichtsprozent) wäßriger Lactose-Lösung.

17. Verwendung einer flüssigen Lösung mit Mikrobläschen bestehend aus

1. der Mischung von 0,01 bis 10 Gewichtsprozenten eines Tensides oder Tensidgemisches mit einer wäßrigen oder mit Wasser mischbaren Trägerflüssigkeit und

2. der Mischung von 0,5 bis 50 Gewichtsprozenten einer viskositätserhöhenden Substanz oder eines Substanzgemisches in einer wäßrigen oder mit Wasser mischbaren Trägerflüssigkeit, wobei

3. beide Mischungen getrennt oder vereinigt vorliegen für die Stoßwellen- und Ultraschalltherapie.

18. Verwendung eines Mittels nach Anspruch 17, bestehend aus der Mischung von

1. 0,01 bis 10 Gewichtsprozenten eines Tensides oder Tensidgemisches in einer wäßrigen oder mit Wasser mischbaren Trägerflüssigkeit, die 0,05 bis 5 Gewichtsprozente eines physiologisch verträglichen, carbonsauren Salzes enthält und

2. der Mischung von 0,5 bis 50 Gewichtsprozenten einer viskositätserhöhenden Substanz oder eines Substanzgemisches mit einer wäßrigen oder mit Wasser mischbaren Trägerflüssigkeit, die die dem carbonsauren Salz äquivalente Menge physiologisch verträglicher Säure enthält.

19. Verwendung eines Mittels nach einem der Ansprüche 17 oder 18, enthaltend ein nichtionogenes Tensid, vorzugsweise ein Polyoxyethylenpolyoxypropylen-Polymeres, das gleichzeitig viskositätserhöhend wirkt.

20. Verwendung eines Mittels nach Anspruch 19, enthaltend ein Tensid, das aus Polyoxyethylenpolyoxypropylen-Polymere mit dem Molekulargewicht 6800 bis 8975 oder 16250 oder 13300 oder das aus einem Polyoxyethylenfettsäureester besteht.

21. Verwendung eines Mittels nach einem der Ansprüche 17 oder 18, enthaltend ein Tensid, das aus Polyoxyethylenstearaten oder aus Natriumlaurylsulfat oder Natriumdioctylsulfosuccinat besteht.

22. Verwendung eines Mittels nach Anspruch 17, enthaltend als Trägerflüssigkeit Wasser oder mit Wasser mischbare ein- oder mehrwertige Alkohole, physiologische Elektrolytlösung oder eine Infusionslösung oder deren Gemische.